# EUROPEAN PATENT APPLICATION

(11) **EP 2 281 803 A1**
(43) Date of publication of application: **09.02.2011**
(21) Application number: 09176511.5
(22) Date of filing: 19.11.2009
(51) Int. Cl.: C07C 231/24, C07C 237/46, B01D 61/02

(54) **Improvements in crystallization of 5-amino-N,N'-bis(2,3-dihydroxypropyl)-2,4,6,-triiodo-isophthalamide, an intermediate for synthesizing non-ionic X-ray contrast agents**

(30) Priority: 21.07.2009 US 227084 P
(71) Applicant: GE Healthcare AS, 0401 Oslo (NO)
(72) Inventor: Ingvoldstad, Odd, Einar, 4521 Spangereid (NO)
(74) Representative: Wulff, Marianne Weiby

(57) **Abstract**

This invention relates to the crystallization process of 5-amino-N, N'- bis(2,3-dihydroxypropyl)-2,4,6-triiodo-1,3-benzenedicarboxamide ("Compound B"), an iodinated intermediate in the preparation of non-ionic X-ray contrast agents. The instant process utilizes a nanofiltration system to pass through low molecular weight salt, thereby reducing the solubility of Compound B in the retentate. This process increases supersaturation of the mother liquor and improves crystal growth in the crystallization following the initial one. The process of the present invention is useful in increasing the overall crystallization yield of Compound B in an industrial manufacturing process.

## Description

### TECHNICAL FIELD

This invention relates generally to non-ionic X-ray contrast agents. It further relates to an improved method in crystallization of an intermediate in the synthesis of non-ionic X-ray contrast agents. In particular, it relates to crystallizing 5-amino-N, N'-bis(2,3-dihydroxypropyl)-2,4,6-triiodo-1,3-benzenedicarboxamide ("Compound B") using a nanofiltration system to increase the crystallization yield.

### BACKGROUND OF THE INVENTION

Non-ionic X-ray contrast agents constitute a very important class of pharmaceutical compounds produced in large quantities. 5-[N-(2,3-dihydroxypropyl)-acetamido]-N,N'-bis(2,3-dihydroxypropyl)-2,4,6-triiodo-isophthalamide ("iohexol"), 5-[N-(2-hydroxy-3-methoxypropyl)acetamido]-N,N'-bis(2,3-dihydroxypropyl)-2,4,6-triiodo-isophthalamide ("iopentol") and 1,3-bis(acetamido)-N,N'-bis[3,5-bis(2,3-dihydroxypropyl-aminocarbonyl)-2,4,6-triiodophenyl]-2-hydroxypropane ("iodixanol") are important examples of such compounds. They generally contain one or two triiodinated benzene rings.

The industrial production of non-ionic X-ray contrast agents involves a multistep chemical synthesis. To reduce the cost of the final product, it is critical to optimize the yield in each step. Even a small increase in yield can lead to significant savings in a large scale production. In particular, iodine is the most expensive reagent in the process. It is thus especially important to obtain a high yield with few impurities and minimal wastage for each synthetic intermediate involving an iodinated compound. Furthermore, improved purity of a reaction intermediate, especially at the latter stage of synthesis, is essential in providing a final drug substance fulfilling regulatory specification such as those expressed on US Pharmacopeia.

In addition to economic and regulatory concerns, the environmental impact of an industrial process is becoming an increasingly significant consideration in the design and optimization of synthetic procedures.

For example, three main processes are known for the preparation of iodixanol (1,3-bis(acetamido)-N,N'-bis[3,5-bis(2,3-dihydroxypropylaminocarbonyl)-2,4 ,6-triiodophenyl]-2-hydroxypropane), all of which start with 5-nitroisophthalic acid. *See* Scheme 1 below and U.S. Patent No. 6,974,882. In the first process shown below, the following route is used, via the final intermediate 5-acetamido-N,N'-bis(2,3-dihydroxypropyl)-2,4,6-triiodoisophthalamide ("Compound A"):

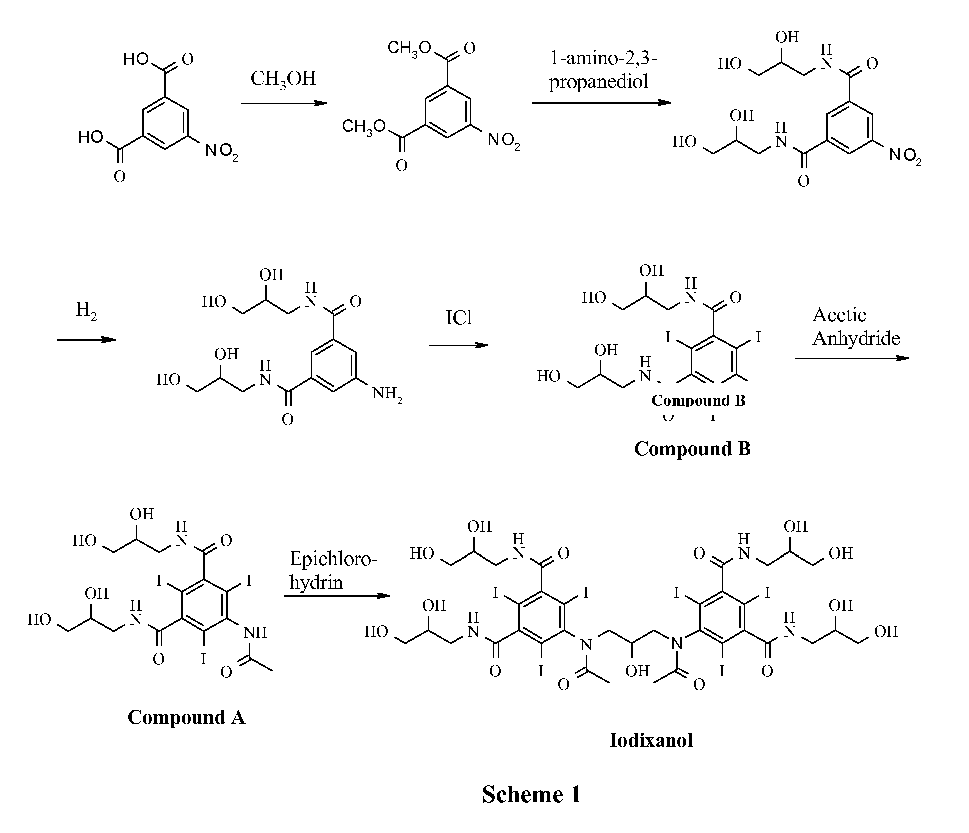

One of the key intermediates in the above synthetic route is the first iodinated compound, 5-amino-N, N'-bis(2,3-dihydroxypropyl)-2,4,6-triiodo-1,3-benzenedicarboxamide ("Compound B"). To achieve the desired purity, Compound B is crystallized before the initiation of the next synthetic step. There exists a need for practical procedures to increase the recovery of Compound B during the crystallization process.

### BRIEF DESCRIPTION OF THE DRAWINGS

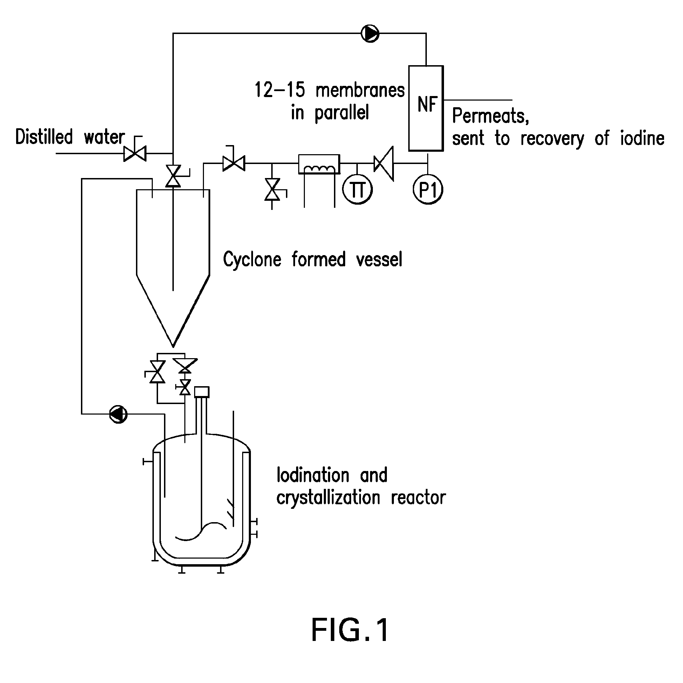

FIG. 1 is a schematic diagram of one exemplary embodiment of the instant invention.

### SUMMARY OF THE INVENTION

The present invention provides a large scale crystallization process of Compound B. This process includes the steps of:
(a) reacting 5-amino-N,N'-bis-(2,3-dihydroxypropyl)isophthalamide with an iodinating agent in an aqueous medium;
(b) crystallizing Compound B from the crude product of the iodination reaction;
(c) feeding the mother liquor of the crystallization suspension of (b) through a nanofiltration assembly whereby the solubility of Compound B in the retentate decreases as salt components permeate through the nanofiltration assembly; and
(d) returning the retentate containing enriched concentration of Compound B to the crystallization suspension of (b).

The instant process considerably increases the crystallization yield of Compound B by about 3%. The minimization of wastage of Compound B also reduces the discharge of UV-absorbing compounds.

### DETAILED DESCRIPTION OF THE INVENTION

To ensure high efficiency synthesis of subsequent intermediates and the final drug products, it is necessary to crystallize compound B. Following the quenching of the iodination reaction, crystals of Compound B are added as seeds for an initial crystallization and the aqueous solution of the crude product of the iodination reaction is chilled. The mother liquor of the initial crystallization suspension is then pumped into a nanofiltration assembly. This system may contain one or more parallel membranes, depending on the scale of production. The nanofiltration allows passage of the low molecular weight species (permeate) to a waste or recovery stream, while retaining the larger molecular weight Compound B on the retentate side of the membrane. The membrane cut-off may be between about 100 and 700 Da, which corresponds to 100-700 g/mole in molecular weight. The suitable temperature may be between about 30-40°C, or in accordance with membrane manufacture specification. A temperature that is too low may cause the undesirable result of precipitating Compound B in the retentate before it re-enters to the crystallization vessel. On the other hand, a temperature that is too high may reduce the supersaturation and hence lower the yield of the process. An elevated temperature may also result in the loss of Compound B through the membrane.

The low molecular weight species include salt components such as NaCl and NaI.

We have surprisingly found that in the instant process the solubility of Compound B depends on the salt concentration. As salt components including NaCl and NaI are removed from the retentate, the solubility of Compound B decreases, which in turn increases the supersaturation of Compound B in the retentate. When the retentate is returned to the initial crystallization suspension, the concentration of Compound B in the mother liquor is further increased. This heightened supersaturation of the mother liquor facilitates the crystal growth in the subsequent stages of crystallization of Compound B. The process of feeding the mother liquor through the nanofiltration assembly can be repeated multiple times in order to further enrich the concentration of Compound B in the mother liquor.

The invention is illustrated further by the following examples that are not to be construed as limiting the invention in scope to the specific procedures described in them.

### EXAMPLES

### EXAMPLE 1 (COMPARATIVE)

5-amino-N, N'-bis(2,3-dihydroxypropyl)-1,3-benzenedicarboxamide (452 kg) is triiodinated with aqueous iodine chloride in an aqueous reaction medium. Excess iodine chloride is quenched by addition of sodium bisulphite. Seeds of 5-amino-N, N'-bis(2,3-dihydroxypropyl)-2,4,6-triiodo-1,3-benzenedicarboxamide (Compound B) (5.5 kg) are added at 80°C. The total amount of water in the mixture at this stage is about 3200-3300 liter. The seeded mixture is slowly cooled to 32°C. Crystal growth is allowed to proceed for 12 hours before filtration and wash of the filter cake with water.

### EXAMPLE 2

5-amino-N,N'-bis(2,3-dihydroxypropyl)-1,3-benzenedicarboxamide (452 kg) is triiodinated with aqueous iodine chloride in an aqueous reaction medium. Excess iodine chloride is quenched by addition of sodium bisulphite. Seeds of 5-amino-N,N'-bis(2,3-dihydroxypropyl)-2,4,6-triiodo-1,3-benzenedicarboxamide (Compound B) (5.5 kg) are added at 80°C. The total amount of water in the mixture at this stage is about 3200-3300 liter. The seeded mixture is slowly cooled to 35°C and subjected to nanofiltration followed by diafiltration with water. The mother liquor is decanted from the slurry and concentrated in the nanofiltration unit to about 60 % of its original volume before it is returned to the crystallization vessel. Crystal growth is allowed to proceed for 12 hours before filtration and wash of the filter cake with water. The isolated yield of Compound B is about 3.5% higher than in comparative example 1.

### EXAMPLE 3

5-amino-N,N'-bis(2,3-dihydroxypropyl)-1,3-benzenedicarboxamide (452 kg) is triiodinated with aqueous iodine chloride in an aqueous reaction medium. Excess iodine chloride is quenched by addition of sodium bisulphite. Seeds of 5-amino-N,N'-bis(2,3-dihydroxypropyl)-2,4,6-triiodo-1,3-benzenedicarboxamide (Compound B) (5.5 kg) are added at 80°C. The total amount of water in the mixture at this stage is about 3200-3300 liter. The seeded mixture is slowly cooled to 35°C and subjected to nanofiltration followed by diafiltration with water. The mother liquor is decanted from the slurry and concentrated in the nanofiltration unit before it is returned to the crystallization vessel. This procedure is repeated twice, resulting in an overall volume reduction of the mother liquor of about 50 %. Crystal growth is allowed to proceed for 12 hours before filtration and wash of the filter cake with water. The isolated yield of Compound B is about 4% higher than in comparative example 1.

All patents, journal articles, publications and other documents discussed and/or cited above are hereby incorporated by reference.

## Claims

1. A process for recovering 5-amino-N,N'-bis(2,3-dihydroxypropyl)-2,4,6-triiodo-1,3-benzenedicarboxamide to increase its crystallization yield comprising the steps:
(a) reacting 5-amino-N,N'-bis-(2,3-dihydroxypropyl)isophthalamide with an iodinating agent in an aqueous medium;
(b) crystallizing 5-amino-N,N'-bis(2,3-dihydroxypropyl)-2,4,6-triiodo-1,3-benzenedicarboxamide) ("Compound B") from the crude product of the iodination reaction;
(c) feeding the mother liquor of the crystallization suspension of (b) through a nanofiltration assembly whereby the solubility of Compound B in the retentate decreases as salt components permeate through the nanofiltration assembly; and
(d) returning the retentate containing enriched concentration of Compound B to the crystallization suspension of (b).
